# EUROPEAN PATENT APPLICATION

(11) **EP 3 011 963 A1**
(43) Date of publication of application: **27.04.2016**
(21) Application number: 14813786.2
(22) Date of filing: 30.04.2014
(51) Int. Cl.: A61K 35/74, A61K 35/66, A61P 17/00, A61P 21/00

(54) **COMPOSITION COMPRISING FILLER AND BOTULINUM TOXIN FOR ALLEVIATING SKIN WRINKLES OR AGING OR TREATING NEUROMUSCULAR DISEASES**

(30) Priority: 20.06.2013 KR 20130070765
(71) Applicant: Seoul National University R&DB Foundation, Gwanak-gu Seoul 08826 (KR)
(72) Inventor: CHOI, Tae Hyun, Seoul 151-057 (KR); PIAO, Ying-lan, Seoul 110-799 (KR); KIM, Suk Wha, Seoul 133-758 (KR); HUR, Woojune, Seoul 140-131 (KR); CHOI, La Mee, Seoul 136-797 (KR); MIN, Hye Jeong, Seoul 110-844 (KR)
(74) Representative: von Kreisler Selting Werner - Partnerschaft von Patentanwälten und Rechtsanwälten mbB
(86) International application number: PCT/KR2014/003836
(87) International publication number: WO 2014/204090

(57) **Abstract**

The present invention discloses a composition comprising a filler and Botulinum toxin for alleviating skin wrinkles or aging or treating neuromuscular diseases. The composition according to the present invention resolves neuromuscular diseases, aging or skin wrinkles caused by two types of causative factors through a single injection. At the same time, the composition according to the present invention has a synergistic effect by which one component improves the efficacy of another component. Therefore, the number of injections is decreased to thereby enhance the convenience of patients and reduce costs.

## Description

### [Technical Field]

The present invention relates to a composition comprising a filler and a botulinum toxin for improving skin wrinkles, preventing skin aging, or treating neuromuscular diseases.

### [Background Art]

In general, facial wrinkles are largely classified into static wrinkles (static rhytides) and dynamic wrinkles (dynamic rhytides). The static rhytides are typically shallow, and are caused by skin aging and volume deficits. For treatment of the static rhytides, the volume deficits of a tissue are filled with a filler. In contrast, the dynamic rhytides are typically deep, and are caused by contraction of facial muscles. For treatment of the dynamic rhytides, a botulinum toxin, for example, BOTOX^{®} as a product name is injected into the facial muscles to paralyze the muscles.

Korean Patent Laid-Open Publication No. 10-2011-0062277 discloses a cosmetic composition of a two-form type multi-pore patch for skin improvement using lyophilization, capable of improving stability of a skin care active component and not being separated from the skin after a long adhesion time.

U.S. Patent Application Publication No. 2009/0196928 discloses a biocompatible hydrogel composition, which forms a hydrogel by a cross-linking reaction using an electrophilic material and a nucleophilic material, and is used for releasing active components such as Botox.

A filler may be used in combination with botulinum toxin to improve skin wrinkles. In this regard, it is known that a filler is generally absorbed within 6 months to 2 years, and movement of muscle promotes such absorption. On the other hand, since the Botox has about 4 to 6 months of action duration, repeated injections are required. When the filler and Botox are separately used according to the related art, inconvenience is increased due to the repeated injections. Therefore, it is required to develop a product having a synergistic effect by complementing mutual functions of the filler and the Botox and decreasing the number of injections to enhance convenience.

### [Disclosure]

### [Technical Problem]

An object of the present invention is to provide a composition comprising a filler and a botulinum toxin for improving skin wrinkles, preventing skin aging, or treating neuromuscular diseases, capable of solving the skin wrinkles and aging caused by two types of causative factors through a single injection and having a synergistic effect in which one component improves efficacy of another component to thereby enhance convenience.

### [Technical Solution]

In order to achieve the foregoing objects, the present invention provides a composition comprising a filler and a botulinum toxin for improving skin wrinkles, preventing skin aging, and/or treating neuromuscular diseases, or a cosmetic composition including the same. The composition of the present invention may be used for beauty/cosmetic or treatment in a subject using the composition, in accordance with specific desired objects. Various fillers known in the art may be used, and a short term filler, a medium term filler or a long term filler may be used depending on continuity. For example, the filler may include at least one selected from the group consisting of hyaluronic acid-based fillers, collagen-based fillers, calcium hydroxy apatite-based fillers and polyacrylamide-based fillers, but the present invention is not limited thereto.

Botulinum toxins known in the art are used as the botulinum toxin of the present invention. For example, the botulinum toxin may include Botox^{®}, Meditoxin^{®}, Dysport^{®}, Botulax^{®}, Myobloc^{®}, BTX^{®}, Xeomin^{®}, and the like, but the present invention is not limited thereto.

The botulinum toxin and the filler may be included at various ratios in the composition of the present invention in consideration of desirable effects, for example, the duration time of effect, and the like. In an exemplary embodiment of the present invention, the composition may comprise about 0.01 to 1 ml of the filler and about 1 to 5,000 unit of the botulinum toxin, based on 1 ml of the composition.

In addition, the composition may comprise about 1 to 5,000 unit of Botox, particularly, about 5 to 2,500 unit of Botox based on 1 ml of the filler, but the present invention is not limited thereto.

### [Advantageous Effects]

The composition according to the present invention may resolve skin wrinkles caused by two types of causative factors, aging or neuromuscular diseases through a single injection, and may provide a synergistic effect in which one component improves efficacy of another component. Therefore, the number of injections may be decreased to thereby enhance the convenience of patients and reduce costs.

### [Description of Drawings]

FIG. 1 shows factors used in calculating sciatic function index (SFI) values.
FIG. 2 shows grade evalutions obtained by scoring grades of rats determined based on the foot shape and the modification degree of rat gaits.
FIG. 3 are pictures showing foot shapes and footprints of rats observed at 4, 8, 11, 13 weeks after injections of 1X Botox (control group), 5X Botox (control group), 5X Botox + 0.1 ml filler, 10X Botox + 0.1 ml filler, and 25X Botox + 0.1 ml filler. In the rats, a standard dose of Botox is defined as 1X, wherein 1X corresponds to 6 unit/kg on the basis of Botox (Allergan), and 1.2 unit per a rat weight of 200g).
FIG. 4 shows footprints of rats obtained after conducting the same experiment as FIG. 3.
FIG. 5 shows SFI values obtained after carrying out the same experiment as FIG. 3.
FIG. 6 is graphs showing numerical values graded based on foot shapes and modification degree of gaits of rats analyzed at 4, 8, 11, 13 weeks after injections of 1X Botox (control group), 5X Botox (control group), 5X Botox + 0.1 ml filler, 10X Botox + 0.1 ml filler, and 25X Botox + 0.1 ml filler.
FIG. 7 shows pictures of rats injected by 250X Botox + filler (3 rats), 50X Botox + filler (3 rats), 35X Botox + filler (3 rats), 30X Botox (1 rat), 25X Botox (2 rats), and 10X Botox (1 rat) groups, in order to determine a dose showing toxicity.

### [Best Mode]

The present invention is made on the basis of discovery that, when a filler and a botulinum toxin (Botox) are administered as one composition, a synergistic effect may be provided, and an effect of the Botox and the filler may continue for a long time, such that the number of injections may be decreased to thereby enhance convenience of patients.

Therefore, according to an exemplary embodiment of the present invention, the present invention provides a composition comprising a filler and a botulinum toxin for improving skin wrinkles, preventing skin aging, and/or treating neuromuscular diseases. The improvement of skin wrinkles or the prevention of skin aging means improvement of symptoms of skin accompanying with aging. For example, the improvement of skin wrinkles or the prevention of skin aging includes improvement of skin wrinkles and skin tone, improvement of skin elasticity, and lifting, but the present invention is not limited thereto.

In the present invention, the wrinkles mean all kinds of wrinkles of the body, particularly, facial wrinkles. The facial wrinkles include static wrinkles (static rhytides) and dynamic wrinkles (dynamic rhytides). The static rhytides are typically shallow, and are caused by skin aging and volume deficits. The dynamic rhytides are typically deep, and are caused by contraction of facial muscles. For treatment of the dynamic rhytides, a botulinum toxin is injected into the facial muscles to paralyze the muscles.

The botulinum toxin is secreted by anaerobic bacteria such as *Clostridium botulinum C. butyricum, C. baratii* and *C. argentinense.* There are seven types of the botulinum toxin, among which A-type and B-type botulinum toxins are purified to be used in medical fields. These toxins suppress the secretion of acetylcholine from a motor nerve terminal region, and paralyze corresponding muscles. Various diseases may be treated by the composition of the present invention due to such characteristics. For example, the composition of the present invention may be used for the treatment of various neuromuscular diseases such as eyelid spasms, torticollis (a condition in which the neck unnaturally leans toward one side due to contraction of neck muscles), cervical dystonia, blepharospasm, axillary hyperhidrosis, anal fissure, vaginismus, achalasia, headache disorders including migraine, etc., idiopathic and neurogenic detrusor hyperplasia, focal dystonia, (limbs, jaw joints, vocal cords, etc.), temporomandibular joint disorder, diabetic neuropathy, vocal cord dysfunction, strabismus, chronic neuropathy, facial muscle hypertrophy (masticatory muscles, etc.), detrusor-sphincter dyssynergia, and benign prostatic hyperplasia as well as for cosmetic purposes such as wrinkle treatment, square jaw surgery, improvement of skin aging, but the present invention is not limited thereto.

The filler is a material of lifting wrinkles to be removed by injecting skin tissue components into valleys of the skin wrinkles to fill in volume. Currently, hyaluronic acid and collagen are the most commonly used as the filler. Skin volume is maintained by gel-typed intangible materials such as collagen, elastin, hyaluronic acid, and the like, forming dermal skeleton.

Various commercially available fillers may be used in the composition of the present invention, and may be appropriately selected by a person skilled in the art. For example, the filler may be largely divided into collagen-based fillers, hyaluronic acid-based fillers (for example, Restylane^{®}, Juvederm^{®}, Perfectha^{®}, Elravie^{®}, Yvoire^{®}, Teosyal^{®}, etc.), calcium hydroxyapatite-based fillers (for example, Radiesse^{®}, etc.), polyacrylamide-based fillers (for example, Aquamid^{®}, Bio-Alcamid^{®}, Royamid^{®}, etc.), and poly-L-lactic acid (PLLA)-based fillers (for example, Sculptra^{®}, etc.), etc., depending on the type of components. The filler may also be divided into temporary biodegradable fillers (1 year or less of duration) such as Restylane^{®}, Juvederm^{®}, Teosyal^{®}, Evolence^{®}, etc., semi-permanent biodegradable fillers (1-2 years of duration) such as Reviderm^{®}, Matridex^{®}, Fascian^{®}, etc., and non-degradable permanent fillers (two years or more of duration) such as Artecoll^{®}, Aquamid^{®}, Bio-Alcamid^{®}, etc., based on the duration of effect. Further, the filler may be divided into a volume-enhancing filler (Voluminizer) for enhancing volume and a promoter (Stimulator) for promoting collagen production after injection, in accordance with an action mechanism.

Various botulinum toxins that are commercially available may be used in the composition of the present invention, and a person skilled in the art may appropriately select a botulinum toxin suitable for the present composition.

The botulinum toxin in the present invention may include any known botulinum toxin produced by bacteria or by a recombinant method, or engineered variants or fusion proteins thereof. There are currently seven types of immunologically distinguishable serotypes, i.e., A-type, B-type, C-type, D-type, E-type, F-type, and G-type botulinum toxins, which may be purchased from Sigma-Aldrich and Metabiologics Inc. (Madison, Wisconsin), and are different from each other in terms of the kinds of animal affected thereby and the symptoms of paralysis caused thereby. In particular, A-type and B-type botulinum toxins are usable as therapeutic and/or cosmetic agents. For example, A-type botulinum toxins include Botox^{®} (Onabotulinum toxin A, Allergan Inc., USA), Meditoxin^{®} (Pacific Pharmaceuticals Co., Ltd., Korea), Botulax^{®} (Hugel Pharma, Korea), Xeomin^{®} (Merz Pharmaceuticals, USA), Dysport^{®} (Ipsen, UK), etc., and B-type botulinum toxins include MYOBLOC^{®} (Elan, USA), etc., but the present invention is not limited thereto.

The botulinum toxin in the present invention may alternatively be a botulinum toxin derivative, that is, a compound having botulinum toxin activity but including chemical modification or functional modification as compared to the one before being modified. For example, the botulinum toxin may have deletion, modification or substitution of at least one amino acid as compared to the botulinum toxin before being modified. For example, the botulinum toxin may be modified by improving or enhancing characteristics of the botulinum toxin or reducing side effects thereof. Alternatively, the botulinum toxin may be whole having an activity or a part thereof, or may be used as itself or as a part of a fusion protein. Further, the botulinum toxin may be a precursor of botulinum toxin, which itself may be non-toxic, for example, a form of a nontoxic zinc protease that shows toxicity by proteolysis.

The composition according to the present application may be prepared as a pharmaceutical or cosmetic composition. Methods of preparing the pharmaceutical or cosmetic composition are known in the art, and appropriate technique related to preparation, formulation and additives may be properly selected by a person skilled in the art.

For example, the composition according to the present invention may be prepared by mixing the filler and the botulinum toxin with a carrier, generally, at least one additionally pharmaceutically acceptable aqueous carrier or excipient. For example, the composition may include a pharmaceutically acceptable carrier or diluent such as buffered saline, and other components typically used in a topical pharmaceutical or cosmetic pharmaceutical composition, including dermatologically or pharmaceutically acceptable carrier, vehicle or medium. The expression "dermatologically or pharmaceutically acceptable" used herein means that excessive toxicity, incompatibility, allergic response, etc., are not caused when the above-described composition or component is in contact with the skin.

For example, the cosmetic composition may include conventional adjuvants and carriers such as antioxidants, stabilizers, solubilizers, vitamins, pigments and/or flavoring agents.

The cosmetic composition of the present invention may contain a water-soluble base material including unsaturated fatty acids such as conjugated linoleic acid, linoleic acid, gamma-linoleic acid and alpha-linoleic acid, or propylene glycol.

Further, the cosmetic composition of the present invention may be prepared in any formulation that is conventionally made in the art, for example, solutions, suspensions, emulsions, pastes, gels, creams, lotions, powders, soaps, surfactant-containing cleansing, oil, powder foundation, emulsion foundation, wax foundation and spray, etc., but the present invention is not limited thereto. More specifically, the cosmetic composition of the present invention may be prepared in flexible skin lotion, nutrition lotion, nutrition cream, massage cream, essence, eye cream, hair tonic, shampoo, rinse, cleansing cream, cleansing foam, cleansing water, pack, spray or powder formulations.

When the composition of the present invention is formulated into a paste, cream or gel, animal oil, vegetable oil, wax, paraffin, starch, tragacanth, cellulose derivative, polyethylene glycol, silicone, bentonites, silica, talc, zinc oxide, or the like, may be used as a carrier component.

When the composition of the present invention is formulated into a powder or spray, lactose, talc, silica, aluminum hydroxide, calcium silicate, or polyamide powders may be used as a carrier component. In particular, when the composition of the present invention is formulated into a spray, propellants such as chlorofluoro hydrocarbon, propane/butane, and dimethyl ether may be additionally contained in the composition.

When the composition of the present invention is formulated into a solution or emulsion, a solvent, a solubilizer, or an emulsifying agent is used as a carrier component. For example, water, ethanol, isopropanol, ethyl carbonate, ethyl acetate, benzyl alcohol, benzyl benzoate, propylene glycol, 1,3-butyl glycol, glycerol aliphatic ester, polyethylene glycol or fatty acid ester of sorbitan may be used.

When the composition of the present invention is formulated into a suspension, liquid diluents such as water, ethanol, and propylene glycol; suspensions such as ethoxylated isostearyl alcohol, polyoxyethylene sorbitol ester, and polyoxyethylene sorbitan ester; microcrystalline cellulose, aluminum meta-hydroxide, bentonite, agar, or tragacanth may be used as a carrier component.

When the formulation of the composition of the present invention is surfactant-containing cleansing, aliphatic alcohol sulfate, aliphatic alcohol ether sulfate, sulfosuccinate monoester, isethionate, imidazolium derivative, methyl taurate, sarcosinate, fatty acid amide ether sulfate, alkyl amido betaine, aliphatic alcohol, fatty acid glyceride, fatty acid diethanolamide, vegetable oil, lanolin derivative, ethoxylated glycerol fatty acid ester, or the like, may be used as a carrier component. According to preferable administration type, the composition of the present invention may include at least one pharmaceutically acceptable excipient.

Further, in each formulation of the cosmetic composition, other components other than essential components may be selected and mixed by a person skilled in the art without any technical difficulty in accordance with the purpose of use, etc.

In addition, the composition according to the present invention may include at least one pharmaceutical adjuvant selected from the group consisting of thickeners, preservatives, fragrances, coloring agents, chemicals or micronutrients filters, moisturizing agents, and thermal spring water, which are known to a person skilled in the art. For example, the preservatives commonly used in cosmetic or functional food may include molecules having antibacterial activity such as caprylic derivatives (capryloyl glycine and glyceryl caprylate), hexane diol and sodium levulinate, zinc and copper derivatives (gluconate and PCA), phytosphingosine and derivatives thereof, benzoyl peroxide, piroctone olamine, zinc pyrithion, selenium sulfide, econazole, ketoconazole, or local antibiotics such as erythromycin and clindamycin.

In addition, if necessary, the composition of the present invention may further include any component that is commonly used for cosmetic care, e.g., for the prevention of skin aging such as wrinkles, or for the treatment of neuromuscular diseases.

In terms of formulation, the composition of the present invention may include solutions, emulsions, suspensions, creams, lotions, gels, powders, or other typical solid or liquid compositions that are suitable for being used in the skin.

In the composition of the present invention comprising the filler and the botulinum toxin, the Botox (on the basis of Allergan) may be contained in an amount of about 1 to 5,000 unit based on 1 ml of the filler, but the present invention is not limited thereto. Accordingly, a person skilled in the art may appropriately select the amount and the ratio of components in consideration of the degree of symptoms requiring improvement or treatment, patient's condition, duration of treatment, etc.

In another exemplary embodiment, the present invention relates to a sustained dosage form comprising Botox as an active ingredient. The composition according to the present invention comprising a filler in combination with a botulinum toxin may resolve two types of wrinkles by a single injection and have a synergistic and lasting effect for efficacy of each component. It is known that the filler is absorbed between about 6 months to 2 years and movement of muscles promotes the absorption. Botox is required to be repeatedly injected since it has an action duration of about 4 to 6 months. However, the composition of the present invention suppresses the movement of facial muscles, such that the survival time of the filler may be increased, and the botulinum toxin is slowly released while absorbing the filler, thereby providing extended effects. Accordingly, the composition of the present invention may have an additional synergistic effect surpassing a mere aggregation of effects of the individual components, reduce the number of injections to enhance convenience of the patient, and reduce side effects and costs.

Hereinafter, preferred embodiments of the present invention will be described to assist in understanding the present invention. However, the following exemplary embodiments are provided only to more easily understand the present invention. The present invention is not limited thereto.

### Example

### Example 1. Preparation of mixed compositions comprising Botox and filler

Spague-Dawley rats (6-week-old, 0.2 kg, Orientbio Inc.) were used for animal experiments, and the Botox used for experiments was made by dissolving Botox 200 unit/vial (Botulax, Hugel Pharma, Korea) having a freeze-dried state in 0.6 ml of saline water. The filler was purchased from Hugel Pharma, Korea. 200 µl of the Botox solution was mixed with 100 µl of the filler. The standard dose of Botox used in the SD rats was determined as "1X (corresponding to 6 unit/kg on the basis of Botox (Allergan), and 1.2 unit per a rat weight of 200g)" (Jianjun Ma et al., Time Course of Recovery of Juvenile Skeletal Muscle After Botulinum Toxin A Injection. Am. J. Phys. Med. Rehabil. Vol.83, No. 10). The rats were divided into 1X Botox control group, 5X Botox control group, 5X Botox + 0.1 ml filler group, 10X Botox + 0.1 ml filler group, and 25X Botox + 0.1 ml filler group, and ratios of Botox and filler injected into the rats were summarized in Table 1 below.

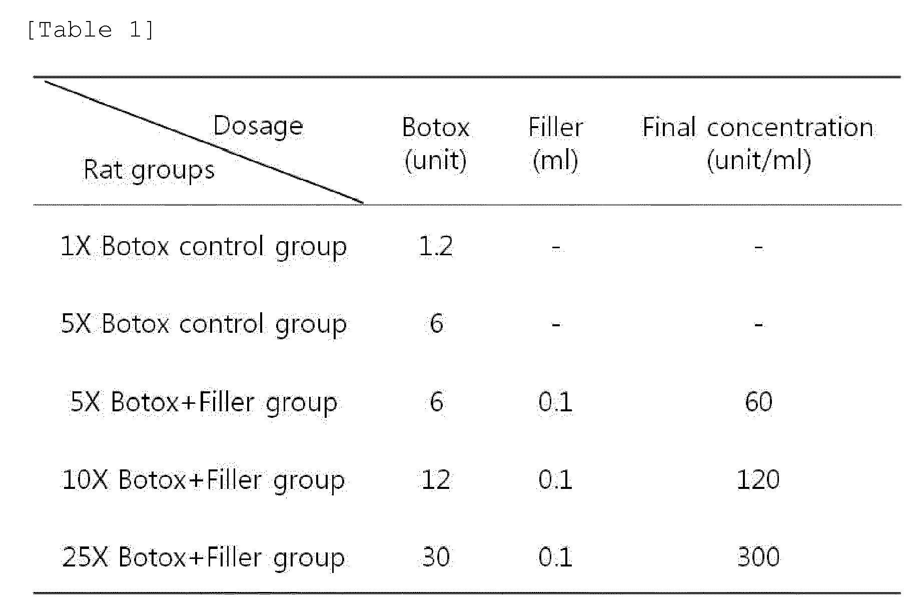

The Botox, and the mixture of Botox and filler injected into the rats had a total volume of 300 µl. The Botox and the mixture of Botox and filler were injected into right gastrocnemius of the anesthetized rats.

In order to evaluate the effect of the Botox and filler, foot shape and footprint of the rats were observed at 4, 8, 11, and 13 weeks after injections, and sciatic function index (SFI) was calculated in accordance with the method described in the reference (Chen, C.J. et al., Transplantation of Bone Marrow Stromal Cells for Peripheral Nerve Repair, Exp. Neurol. 2007; 204:443-53).

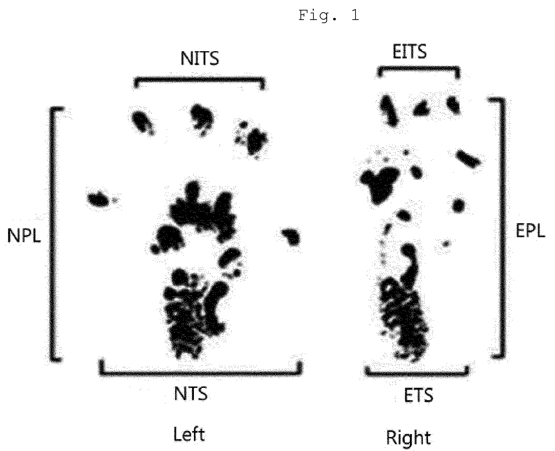

Specifically, the footprints were observed by applying ink to feet of the rats and allowing the rats to walk on white paper several times. Here, a length between a third toe and heel is PL, a length between a first toe and a fifth toe is TS, and a length between a second toe and a fourth toe is ITS. The SFI values were obtained by substituting the footprints of right feet (EPL, ETS, EITS) into which Botox is injected and the footprints of normal left feet (NPL, NTS, NITS) in the Equation, respectively (for general calculation, see the following Equation, FIG. 1 and Exp. Neurol. 2007; 204:443-53). SFI=-38.3×(EPL-NPL)/NPL+109.5×(ETS-NTS)/NTS+13.3×(EITS-NITS)/NITS-8.8.

### Example 2. Analysis of effect of mixed compositions comprising Botox and filler

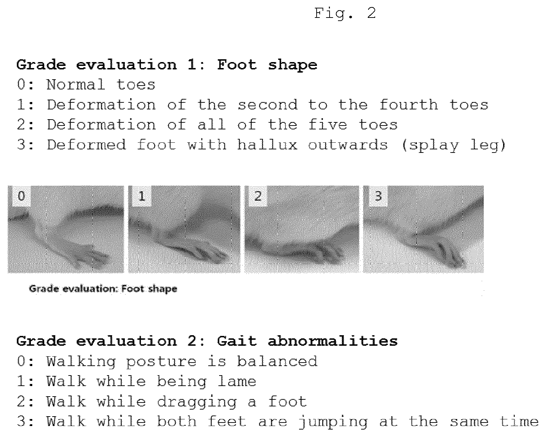

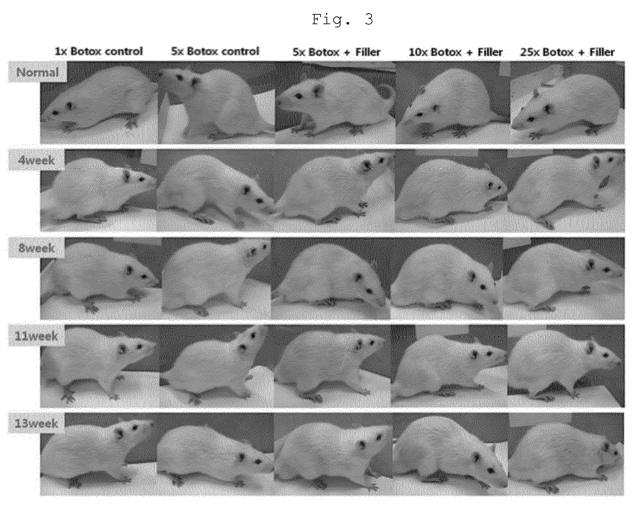

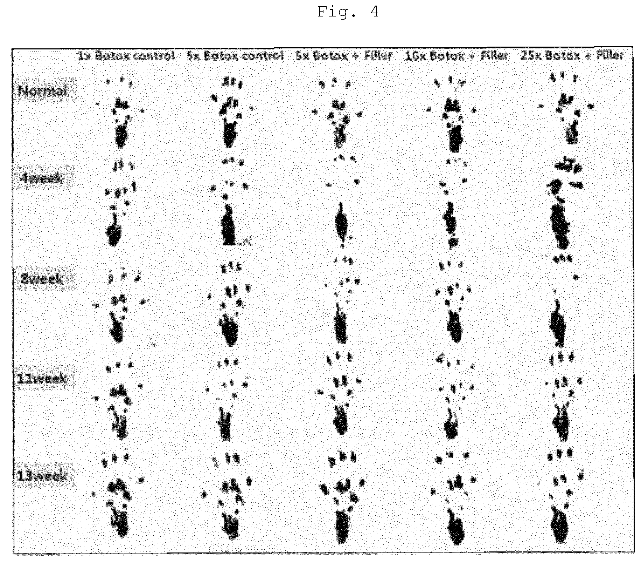

Analysis results obtained from Example 1 were shown in FIGS. 2 to 6. The SFI values were almost 0 when the rat has normal function of gastrocnemius, and as the SFI value becomes close to -100, overall dysfunction of gastrocnemius was shown. In the present experiment, as the SFI value becomes close to -100, the effect of Botox was better. After 13 weeks for the experiment, the effect of Botox was the best at 4 weeks in all groups, and as time went by, the effect of Botox was slightly reduced. In the Botox + filler group, the SFI values were increased, which had more lasting Botox effect, as compared to the group injected with Botox only. Specifically, in 1X Botox control group, the SFI value was -9.5 at 8 weeks, which is almost a level of a normal rat (the normal SFI value is about -8.8). In 5X Botox control group, the SFI value was increased by 72.74% (from -63.86 at 4 weeks to -17.41 at 13 weeks), showing that the effect of Botox was rapidly reduced. Meanwhile, in the mixed compositions comprising Botox and filler, the SFI value was increased by 62.56% (-61.79 at 4 weeks to -23.13 at 13 weeks) in 5X Botox + 0.1 ml filler group, the SFI value was increased by 55.17% (-61.14 at 4 weeks to -27.41 at 13 weeks) in 10X Botox + 0.1 ml filler group, and the SFI value was increased by 56.53% (-89.29 at 4 weeks to -38.81 at 13 weeks) in 25X Botox + 0.1 ml filler group. It indicates that the effect of Botox was highly maintained at 13 weeks in these three experimental groups as compared with 1x and 5X Botox control groups (see FIGS. 3, 4 and 5).

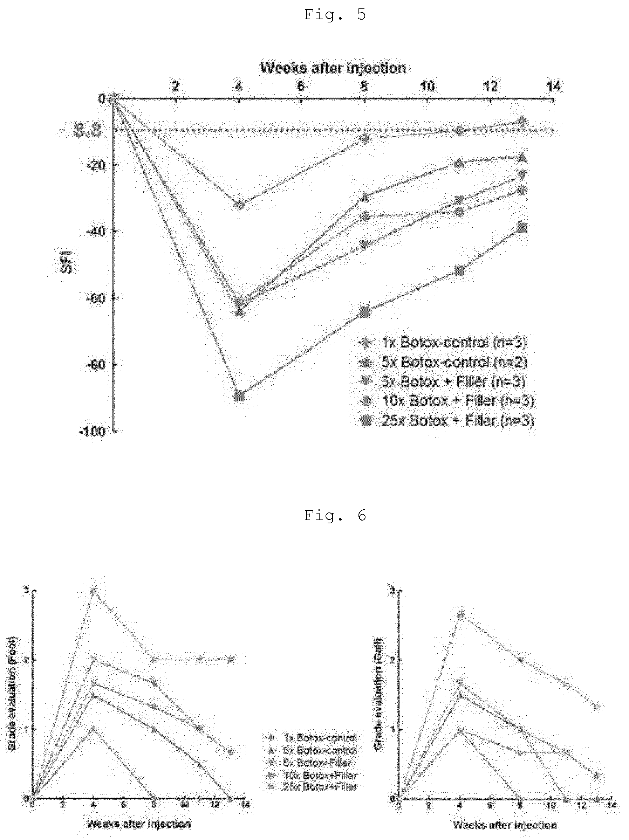

Grade evaluation sheets 1 and 2 (*see* FIG. 2) were made depending on the modification degree of right foot shapes and gaits of each group into which Botox, or Botox + filler is injected, and each score was calculated. In the grade evaluation sheets of the present experiment, the higher score indicates that the foot shape of the rats is more severely modified (Grade evaluation sheet 1), and the gait becomes more imbalanced (Grade evaluation sheet 2). Further, the higher the score is, the higher the effect of Botox and filler is. As results obtained by injecting Botox, or Botox + filler, and conducting grade evaluation using Grade evaluation sheets from 4 weeks to 13 weeks, it was confirmed that, in 25X Botox + 0.1 ml filler group, 10X Botox + 0.1 ml filler group and 5X Botox + 0.1 ml filler group, the modification of foot shape was severe at 13 weeks as compared to 1X and 5X Botox control groups, and among them, 25X Botox + 0.1 ml filler group had the highest score in the grade evaluation. At 4 weeks, all groups had the most severe modification of foot shape and had high level, and as time went by, the grades were getting low. In the Grade evaluation sheet 1 (foot shape), 1X and 5X Botox control groups already had normal grade at 8 and 11 weeks, that is, 0 point; on the other hand, 25X Botox + 0.1 ml filler group had distinct modification in the foot shape and maintained 2 points (the highest score was 3 points) even at 13 weeks (*see* FIG. 6).

It means that, in the mixed composition of filler and Botox, the effect of Botox was continuously maintained for a long time as compared to the group injected with Botox alone. This result demonstrates that the combination of Botox and filler could achieve an additional synergistic effect surpassing a mere aggregation of effects of the individual components.

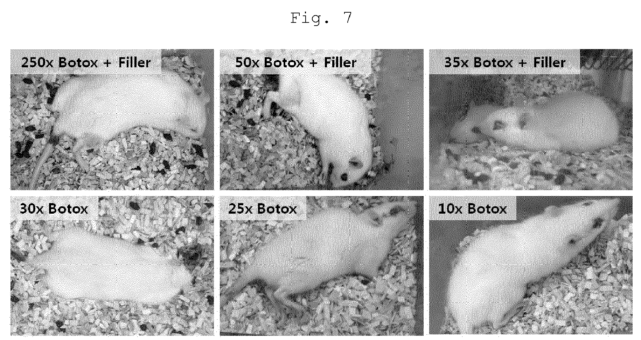

Further, in order to determine a dose showing toxicity, Botox and the filler were injected into 250X Botox + filler group (3 rats), 50X Botox + filler group (3 rats), 35X Botox + filler group (3 rats) as additional experiments, and Botox alone was injected into 30X Botox group (1 rat), 25X Botox group (2 rats), and 10X Botox group (1 rat). As a result, all rats were dead except for 10X Botox group (see Table 2 and FIG. 7). In particular, the rats of 25X Botox group were all dead, but the rats into which 25X Botox + 0.1 ml filler was injected were normally raised well, which means that by mixing Botox and the filler, the toxin of Botox slowly released without giving harmful effects to the animal at a time.

**[Table 2]**

| **Groups** | **Injected dose** | | **Experimental rats** | **dead** |
|---|---|---|---|---|
| | **Botox (unit)** | **Filler (ml)** | | |
| **250X Botox+Filler** | **300** | **0.05** | **3** | **3** |
| **50X Botox+Filler** | **60** | **0.05** | **3** | **3** |
| **35X Botox+Filler** | **42** | **0.1** | **3** | **3** |
| **30X Botox** | **36** | **0** | **1** | **1** |
| **25X Botox** | **30** | **0** | **2** | **2** |
| **10X Botox** | **12** | **0** | **1** | **0** |

While this invention has been described in connection with what is presently considered to be practical exemplary embodiments, it is to be understood that the invention is not limited to the disclosed embodiments, but, on the contrary, is intended to cover various modifications and equivalent arrangements included within the spirit and scope of the appended claims.

Unless defined otherwise, all technical terms used in the present invention have the same meanings generally understood by those skilled in the art to which the present invention pertains. Each reference cited in the present invention is incorporated herein by reference in its entirety.

## Claims

1. A composition comprising a filler and a botulinum toxin for improving skin wrinkles, preventing skin aging, or treating neuromuscular diseases.

2. The composition of claim 1, wherein the filler is a short term filler, a medium term filler or a long term filler.

3. The composition of claim 1, wherein the filler is at least one selected from the group consisting of hyaluronic acid-based fillers, collagen-based fillers, calcium hydroxy apatite-based fillers and polyacrylamide-based fillers.

4. The composition of claim 1, wherein the botulinum toxin is Botox^{®}, Meditoxin^{®}, Dysport^{®}, Botulax^{®}, Myobloc^{®}, BTX^{®} or Xeomin^{®}.

5. The composition of claim 1, wherein the composition comprises 0.01 to 1 ml of the filler and 1 to 5,000 unit of the botulinum toxin, based on 1 ml of the composition.

6. The composition of claim 1, which comprises 1 to 5,000 unit of Botox based on 1 ml of the filler.

7. The composition of claim 6, which comprises 5 to 2,500 unit of Botox based on 1 ml of the filler.

8. The composition of claim 1, wherein the effect of the botulinum toxin is maintained for a long time as compared to a composition without a filler.

9. The composition of claim 1, wherein the neuromuscular diseases is eyelid spasms, torticollis, cervical dystonia, blepharospasm, axillary hyperhidrosis, anal fissure, vaginismus, achalasia, headache disorders including migraine, idiopathic and neurogenic detrusor hyperplasia, focal dystonia, temporomandibular joint disorder, diabetic neuropathy, vocal cord dysfunction, strabismus, chronic neuropathy, facial muscle hypertrophy, detrusor-sphincter dyssynergia, and benign prostatic hyperplasia.

10. A cosmetic composition comprising a composition according to any of claims 1 to 9.
